# EUROPEAN PATENT SPECIFICATION

(11) **EP 1 164 949 B1**
(45) Date of publication and mention of the grant of the patent: **02.05.2003**
(21) Application number: 00917479.8
(22) Date of filing: 30.03.2000
(51) Int. Cl.: A61B 17/32, A61B 17/42

(54) **SURGICAL CUTTING INSTRUMENT**
CHIRURGISCHES SCHNEIDEINSTRUMENT
INSTRUMENT DE COUPE CHIRURGICAL

(30) Priority: 30.03.1999 NL 1011704
(43) Date of publication of application: 02.01.2002
(73) Proprietor: Espro B.V., 3403 ZS IJsselstein (NL)
(72) Inventor: VEERSEMA, Sebastiaan, NL-3403 ZS Ijsselstein (NL)
(74) Representative: van Westenbrugge, André
(86) International application number: NL0000211
(87) International publication number: WO00057798

(56) References cited:
- DE-A- 2 928 689
- GB-A- 638 892
- US-A- 3 631 858
- US-A- 3 877 145
- US-A- 4 753 636

## Description

The application relates to a surgical cutting instrument, comprising a handle and a blade connected thereto. Scissors are usually used for an episiotomy, an obstetric operation in which during the confinement a cut is made in the vagina of the woman giving birth, in order to create more space, so that tearing of the vagina is prevented or the delivery is speeded up. When performing the episiotomy, the obstetrician (general practitioner, midwife or gynaecologist) inserts the fingers of the left hand into the vagina in order to protect the baby's head and to guide the scissors while the scissors are being operated with the right hand. The incision made with the scissors, to a length of approximately 4.5 cm, must be made in one go, in order to obtain a clean and straight incision. Generally when the obstetrician is making the incision, he or she takes up a position on the right-hand side of the patient, while the cutting direction for a right-handed obstetrician is towards the left for making the incision. If the obstetrician is left-handed, he or she will have problems in operating the right-handed scissors, with the result that cutting will be more difficult. Apart from the complexity of the known episiotomy, for which two hands are needed, the known method has the further disadvantage that the scissors can become blunt through handling procedures such as sterilization. Moreover, the cutting noise of the known scissors can be traumatic for the patient or for members of the family present at the confinement.

A pertinent example of the prior art, i.e. scissors having a smooth tapered flange, integral with a lower blade, which may be operated with one hand, is illustrated in the publication US-A-3 877 145.

It is therefore an object of the present invention to provide a surgical cutting instrument, in particular an obstetric cutting instrument, which can be put into position in a safe and reliable manner by right-handed and left-handed persons, and which can be operated very easily with one hand. It is a further object of the present invention to provide a cutting instrument by means of which a regular cut with an accurately controllable length can be obtained.

To this end, the surgical cutting instrument according to the present invention is characterized in that the handle comprises a bottom lip and a top blade-holder connected thereto, an elongate accommodation area being formed between the lip and the blade-holder, for the accommodation of tissue to be cut through, in which the blade is accommodated in such a way that it is movable inside the blade-holder and can be placed with a cutting edge against the bottom lip through a gap in the side of the blade-holder facing the lip, and on an external side facing away from the gap the blade-holder is provided with a control element which acts upon the blade.

In the retracted position the blade is fully inside the blade-holder, and the tissue of the vagina to be cut through can be placed in the correct position in the accommodation area. After correct positioning of the cutting instrument, operation of the control element, which can comprise, for example, a spring pawl, will cause the blade to be taken into the accommodation area, so that the cutting edge of the blade is pressed through the tissue, with the result that an incision is obtained in one smooth movement. During use, the cutting instrument can be held with one hand between thumb and index finger or between thumb and index and middle finger, and taken into position, and the blade can then be moved into the cutting position, after which the cutting instrument is withdrawn in the closed position from the vagina. In this way an accurate incision is made in one go in a rapid and simple manner, while the left-handed or right-handed obstetrician can cut either towards the left or towards the right. The cutting instrument according to the invention has the further advantages that it does not have any sharp or pointed parts which could lead to injuries before, during or after use, and that disturbing cutting noises are reduced.

In one embodiment of a cutting instrument according to the invention the control element can be hinged to the blade-holder. The control element can comprise a push arm which acts upon a rear side of a blade, for example a scalpel, which is moved out of the blade-holder by pressing down the control element in a direction crosswise to the cutting edge of the blade.

The blade-holder is preferably hinged to the lip, so that the cutting instrument can be inserted in the open position with the lip into the vagina, which means that when the blade-holder is swung back a good view of the area to be cut through is possible. The blade-holder is then swung to a position above the lip and by operation of the push arm, the blade is pushed against the lip and an incision is obtained.

The blade is preferably placed under spring tension, as a result of which the blade remains placed in an operationally safe manner inside the blade-holder until the control element is depressed against the spring tension. After the incision has been made and the thumb is taken off the control element, the blade is pushed back into the blade-holder again by the spring force, so that the cutting edge is completely enclosed.

The outside of the blade-holder preferably extends at an angle relative to the lip, and the rear part of the lip forms an acute angle with the outside of the blade-holder. This produces a substantially V-shaped external periphery of the cutting instrument, which - because of the fact that the rear side tapers to a point - can be accommodated very easily in the hollow between thumb, index finger and middle finger.

Two embodiments of a cutting instrument according to the present invention will be explained in further detail by way of example with reference to the appended drawing, in which:
Figure 1 shows a perspective view of the use of the cutting instrument according to the present invention;
Figures 2 to 4 show a side view and longitudinal sections respectively of the cutting instrument according to the present invention; and
Figure 5 shows an embodiment of a cutting instrument in which the blade-holder is hinged to the lip.

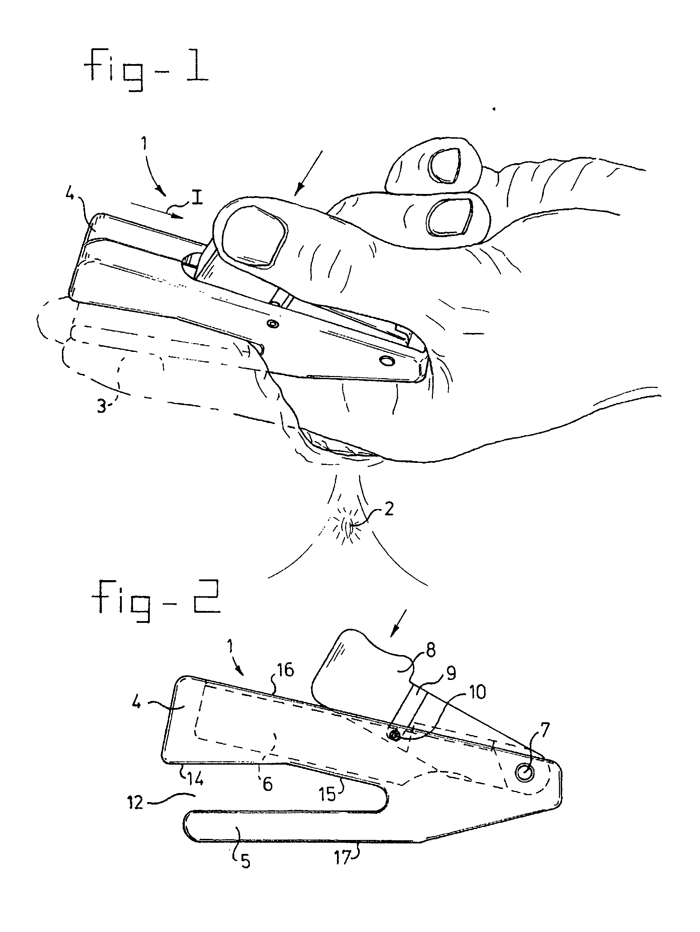

Figure 1 shows a diagrammatic view of the operation of the cutting instrument 1 according to the present invention for obstetric purposes. During use, the cutting instrument is placed by the obstetrician between thumb, index finger and middle finger, the thumb being positioned along the top side of the cutting instrument, and the index and middle fingers extending along the underside 3 of the instrument. The front part of the underside 3 is inserted into the vagina with the index and middle fingers, so that the patient's skin extends into the accommodation area of the instrument. The anus is indicated by reference numeral 2 in Figure 1. Pressing down the thumb then causes a control element 8 to engage with a blade present in the blade-holder 4, which blade is pushed downwards through the tissue of the patient, so that an accurately formed incision is obtained, after which the cutting instrument is withdrawn in the direction of the arrow I, with the blade in the retracted position.

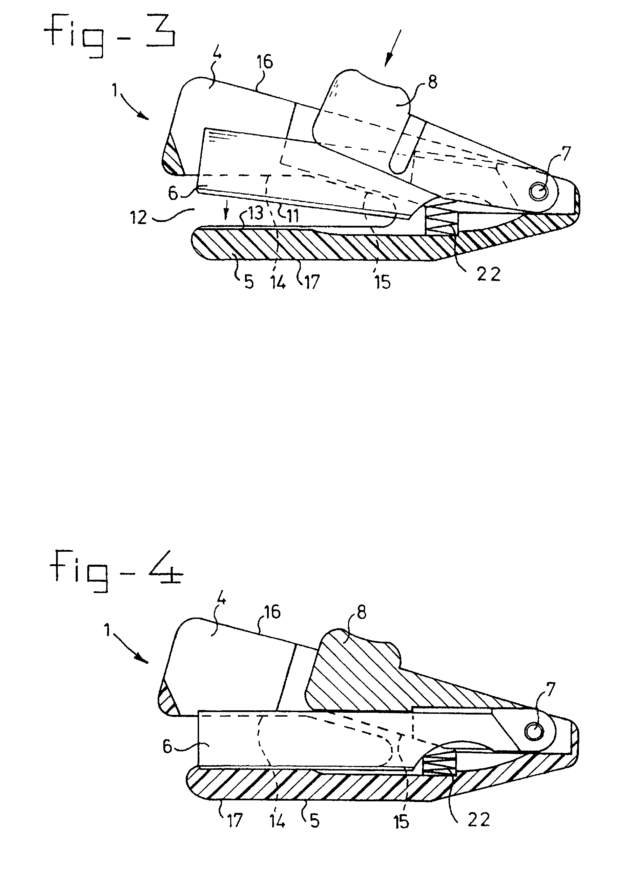

As becomes clear from Figure 2, the cutting instrument 1 comprises an upper blade-holder 4 and a lip 5 situated below it. A blade 6 is hinged to a pin 7 in the blade-holder 4. A push arm 8, which is likewise hinged to the pin 7, acts upon the blade 6. The push arm 8 comprises a slit 9, into which a guide pin 10 fixed to the blade-holder 4 falls, for a stable downward movement of the push arm 8.

As shown in Figure 3, when depressed, the push arm 8 pivots about the pin 7, so that the blade 6 is moved with a cutting edge 11 in the direction of the lip 5. A spring 12 is accommodated between the inside of the lip 5 and push arm 8, in order to prevent the blade 6 from being moved prematurely into the accommodation area 12 of the cutting instrument 1 prior to use, and in order to obtain a stable cutting action when the push arm 8 is depressed. The blade 6 is also pushed back into blade-holder 4 again by the spring element 22, after use.

As can be seen in Figure 4, the cutting edge 11 lies in a slit 13 on the inside of the bottom lip 5 when the blade 6 is in the extreme downward position.

As can be seen clearly from Figures 2 and 3, the front part 14 of the side of the blade-holder 4 facing lip 5 extends parallel to the inside of the lip 5, while the rear part 15 is directed at an angle towards the lip 5. This means that the accommodation area 12 has a tapering rear end in which the patient's skin can be positioned and gripped, so that it will be prevented from slipping after the cutting instrument 1 has been placed in position. Furthermore, an ergonomically optimum shape is obtained through the fact that the outside 16 of the blade-holder 4 and the outside 17 of the lip 5 extend in a substantially V-shape, the rear part of the outside 17 extending at a more acute angle relative to the top side 16 than the front part. As shown in Figure 1, the cutting instrument 1 can consequently be placed easily between thumb and index finger.

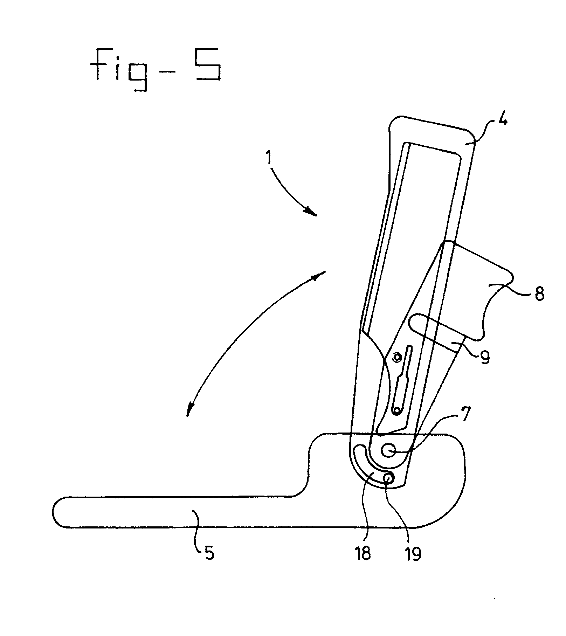

In the embodiment shown in Figure 5, the blade-holder 4 is hinged to the bottom lip 5. The blade-holder 4 is provided with a curved slit 18, into which a hinge pin 19 falls. The shape of the slit 18 ensures that when blade-holder 4 closes in the direction of lip 5 a position in which the blade lies virtually parallel to the lip 5 is obtained, in order to provide an optimum cutting action of the blade 6. The hinged embodiment according to Figure 5 is particularly easy to use, since when it is opened out the cutting device can be moved into the desired orientation, so that a visual inspection of the area of tissue to be cut through is possible. If the cutting device is in the correct position, the blade-holder 4 is swung downwards about the hinge pin 19, and the incision can be made by operation of push arm 8. The blade 6 can be fixed permanently in the blade-holder 4, so that the entire cutting device can be thrown away after one use, or after use a few times. However, it is also possible, in particular in the case of the hinged device according to Figure 5, to fix the blade 6 detachably in the blade-holder 4, so that the blade 6 can be changed after use, while blade-holder 4 and lip 5 fixed thereto can be used several times.

## Claims

1. Surgical cutting instrument (1), comprising a handle, a blade (6) and a bottom lip (5) connected thereto, **characterized in that** the handle comprises a top blade-holder (4) connected thereto, an elongate accommodation area (12) being formed between the lip (5) and the blade-holder (4), for the accommodation of tissue to be cut through, **in that** the blade (6) is movably accommodated in the blade-holder (4) and can be placed with a cutting edge (11) against the bottom lip (5) through a gap in the side of the blade-holder (4) facing the lip (5), and **in that** on an external side (16) facing away from the gap, the blade-holder (4) is provided with a control element (8) which acts upon the blade (6).

2. Surgical cutting instrument (1) according to Claim 1, **characterized in that** the control element (8) is hinged to the blade-holder (4).

3. Surgical cutting instrument (1) according to Claim 1, **characterized in that** the blade-holder (4) is hinged to the lip (5).

4. Surgical cutting instrument (1) according to Claim 1 or 2, **characterized in that** a spring element (22) is placed between the blade (6) and the lip (5).

5. Surgical cutting instrument (1) according to one of the preceding claims, **characterized in that** at a side facing the blade-holder (4) the lip (5) is provided with a slit (13), for the accommodation of the cutting edge (11) of the blade (6).

6. Surgical cutting instrument (1) according to one of the preceding claims, **characterized in that** a front part (14) of the inside of the blade-holder (4) which faces the lip (5) extends substantially parallel to the lip (5), and a rear part (15) of the inside of the blade-holder (4) extends at an angle to the lip (5).

7. Surgical cutting instrument (1) according to one of the preceding claims, **characterized in that** the outside (16) of the blade-holder extends at an angle relative to the lip (5), and **in that** a rear part of the lip (5) forms an acute angle with the outside (16) of the blade-holder (4).

8. Surgical cutting instrument (1), **characterized in that** the blade (6) is accommodated detachably in the blade-holder (4).

## Patentansprüche

1. Chirurgisches Schneidinstrument (1), umfassend einen Handgriff, eine Klinge (6) und eine damit verbundene Bodenlippe (5), **dadurch gekennzeichnet, dass** der Handgriff einen mit ihm verbundenen oberen Klingenhalter (4) umfasst, ein längliches Aufnahmegebiet (12), das zwischen der Lippe (5) und dem Klingenhalter (4) geformt ist, zur Aufnahme des durchzuschneidenden Gewebes, dass die Klinge (6) bewegbar in dem Klingenhalter (4) untergebracht ist und mit einer Schneidkante (11) gegen die Bodenlippe (5) durch einen Spalt in der Seite des Klingenhalters (4), der in Richtung auf die Lippe (5) gerichtet ist, platziert werden kann, und dass auf einer Außenseite (16), die weg von dem Spalt gerichtet ist, der Klingenhalter (4) mit einem Steuerelement (8) versehen ist, das auf die Klinge (6) wirkt.

2. Chirurgisches Schneidinstrument (1) nach Anspruch 1, **dadurch gekennzeichnet, dass** das Steuerelement (8) drehbar am Klingenhalter (4) angebracht ist.

3. Chirurgisches Schneidinstrument (1) nach Anspruch 1, **dadurch gekennzeichnet, dass** der Klingenhalter (4) drehbar an der Lippe (5) angebracht ist.

4. Chirurgisches Schneidinstrument (1) nach Anspruch 1 oder 2, **dadurch gekennzeichnet, dass** ein Federelement (22) zwischen die Klinge (6) und die Lippe (5) platziert ist.

5. Chirurgisches Schneidinstrument (1) nach einem der vorhergehenden Ansprüche, **dadurch gekennzeichnet, dass** an einer Seite, die in Richtung auf den Klingenhalter (4) gerichtet ist, die Lippe (5) mit einem Schlitz (13) zur Aufnahme der Schneidkante (11) der Klinge (6) versehen ist.

6. Chirurgisches Schneidinstrument (1) nach einem der vorhergehenden Ansprüche, **dadurch gekennzeichnet, dass** ein vorderer Teil (14) der Innenseite des Klingenhalters (4), der in Richtung auf die Lippe (5) gerichtet ist, sich im Wesentlichen parallel zur Lippe (5) erstreckt, und ein hinterer Teil (15) der Innenseite des Klingenhalters (4) sich unter einem Winkel zur Lippe (5) erstreckt.

7. Chirurgisches Schneidinstrument (1) nach einem der vorhergehenden Ansprüche, **dadurch gekennzeichnet, dass** die Außenseite (16) des Klingenhalters sich unter einem Winkel relativ zu der Lippe (5) erstreckt, und dass ein hinterer Teil der Lippe (5) einen spitzen Winkel mit der Außenseite (16) des Klingenhalters (4) bildet.

8. Chirurgisches Schneidinstrument (1), **dadurch gekennzeichnet, dass** die Klinge (6) lösbar in dem Klingenhalter (4) aufgenommen ist.

## Revendications

1. Instrument de coupe chirurgical (1), auquel sont connectées une poignée, une lame (6) et une lèvre inférieure (5), **caractérisé en ce qu'**un porte-lame supérieur (4) est connecté à la poignée, une longue zone réceptrice (12) étant formée entre la lèvre (5) et le porte-lame (4) pour recevoir les tissus à découper, **en ce que** la lame (6) est logée de manière mobile dans le porte-lame (4) et peut être placée de façon à avoir un bord de coupe (11) contre la lèvre inférieure (5), à travers un interstice aménagé dans la face du porte-lame (4) qui est en regard de la lèvre (5), et **en ce que** le porte-lame (4) est pourvu, sur une face extérieure (16) dirigée à l'opposé de l'interstice, d'un élément de commande (8) qui agit sur la lame (6).

2. Instrument de coupe chirurgical (1) selon la revendication 1, **caractérisé en ce que** l'élément de commande (8) est articulé au porte-lame (4).

3. Instrument de coupe chirurgical (1) selon la revendication 1, **caractérisé en ce que** le porte-lame (4) est articulé à la lèvre (5).

4. Instrument de coupe chirurgical (1) selon la revendication 1 ou 2, **caractérisé en ce qu'**un élément formant ressort (22) est placé entre la lame (6) et la lèvre (5).

5. Instrument de coupe chirurgical (1) selon l'une quelconque des revendications précédentes, **caractérisé en ce que** la lèvre (5) est pourvue d'une fente (13) au niveau de sa face côté porte-lame (4), pour recevoir le bord de coupe (11) de la lame (6).

6. Instrument de coupe chirurgical (1) selon l'une quelconque des revendications précédentes, **caractérisé en ce qu'**une partie avant (14) de l'intérieur du porte-lame (4), qui est en regard de la lèvre, s'étend d'une manière essentiellement parallèle à la lèvre (5), et une partie arrière (15) de l'intérieur du porte-lame (4) s'étend à un certain angle par rapport à la lèvre (5).

7. Instrument de coupe chirurgical (1) selon l'une quelconque des revendications précédentes, **caractérisé en ce que** l'extérieur (16) du porte-lame s'étend à un certain angle par rapport à la lèvre (5), et **en ce qu'**une partie arrière de la lèvre (5) forme un angle aigu vis-à-vis de l'extérieur (16) du porte-lame (4).

8. Instrument de coupe chirurgical (1), **caractérisé en ce que** la lame (6) est logée de manière détachable dans le porte-lame (4).
